Europäisches Patentamt

European Patent Office

Office européen des brevets

(19)

(11) **EP 1 387 703 B1**

(12) **EUROPEAN PATENT SPECIFICATION**

(45) Date of publication and mention
of the grant of the patent:
**26.07.2006 Bulletin 2006/30**

(51) Int Cl.:
*A61L 31/04* (2006.01)     *A61L 31/14* (2006.01)
*A61K 38/00* (2006.01)

(21) Application number: **01933275.8**

(22) Date of filing: **09.05.2001**

(86) International application number:
**PCT/US2001/015125**

(87) International publication number:
**WO 2002/089868 (14.11.2002 Gazette 2002/46)**

(54) **FIBRIN MATERIAL AND METHOD FOR PRODUCING AND USING THE SAME**

FIBRINMATERIAL UND VERFAHREN ZU SEINER HERSTELLUNG UND VERWENDUNG

MATIERE A BASE DE FIBRINE ET PROCEDE DE PRODUCTION ET D'UTILISATION
CORRESPONDANT

(84) Designated Contracting States:
**AT BE CH CY DE DK ES FI FR GB GR IE IT LI LU
MC NL PT SE TR**

(43) Date of publication of application:
**11.02.2004 Bulletin 2004/07**

(60) Divisional application:
**06075900.8**

(73) Proprietor: **Baxter International Inc.
Deerfield, Illinois 60015 (US)**

(72) Inventors:
• **DELMOTTE, Yves
B-7332 Neufmaison (BE)**
• **DIORIO, James
Antioch, IL 60002 (US)**

(74) Representative: **Bassett, Richard Simon et al
Eric Potter Clarkson LLP
Park View House
58 The Ropewalk
Nottingham NG1 5DD (GB)**

(56) References cited:
WO-A-98/02098          WO-A-99/29338
US-A- 5 318 524          US-A- 5 989 215

**Description**

**Technical Field**

This invention provides a method of producing fibrin hydrogel material and particularly a fibrin hydrogel useful as a drug delivery vehicle and for the prevention of post surgical adhesion.

**Background Art**

One of the major problems in intra-abdominal surgery is the avoidance of post-operative adhesions. It is well-known that adhesions contribute to pain, immobility, retarded wound healing, and in particular to intestinal obstruction which even may be life-threatening. In the field of gynecological surgery, post-surgical adhesions involving female reproductive organs may result in infertility.

Each surgical procedure necessarily produces various forms of trauma where the abdominal cavity or other human cavity is opened for an inspection. Physiologically, the process of wound closure then starts when bleeding ceases upon formation of a hemostatic clot at the places where blood vessels are injured. The clot, at first comprising mainly platelets, is solidified by a fibrin network resulting from the activation of an enzyme cascade involving thrombin, factor XIII and calcium. Further steps on the way to the sealing of the wound are retraction of the hemostatic clot, invasion of various cell types including fibroblasts into the wound area and eventually the lysis of the fibrin network. Adhesions are thought to begin to form when the fibrin clot covering an injury comes into contact with a bleeding adjacent surface and the new connective tissue produced by the fibroblasts attach the two surfaces together.

The problems associated with adhesions often require a further operative procedure for removing/lysing the adhesions, called adhesiolysis, which, like the first operation, principally bears the risk of forming additional adhesions.

Accordingly, the prevention of adhesion formation is medically important. Among the different approaches for prevention of adhesion formation, one involves the use of materials as aphysical or bio-mechanical barrier for the separation or isolation of traumatized tissues during the healing process. Both synthetic materials and natural materials have been used as a barrier to adhesion formation. Permanent, inert implants like Gore Tex® surgical membranes consisting of expanded polytetrafluoroethylene (PTFE) generally require a second operative procedure to remove them, while others such as surgical membranes of oxidized regenerated cellulose are biodegradable, but are thought to elicit an inflammatory response ultimately leading to adhesion formation (A.F. Haney and E. Doty, Fertility and Sterility, 60, 550-558, 1993).

Fibrin sealants and glues are well-known in the art for use in haemostasis, tissue sealing, and wound healing and have been commercially available for more than a decade. Use for anti-adhesion and drug delivery vehicle in glaucoma surgical procedures is one example. Fibrin glues mimic the last step of the coagulation cascade and are usually commercialized as kits comprising two main components. The first component is a solution comprising fibrinogen with or without factor XIII, while the second component is a thrombin calcium solution. After mixing of components, the fibrinogen is proteolytically cleaved by thrombin and thus converted into fibrin monomers. Factor XIII is also cleaved by thrombin into its activated form (FXTIIa). FXIIIa cross links the fibrin monomers to form a three-dimensional network commonly called "Fibrin Gel."

As disclosed in the commonly assigned published PCT patent application, WO 96/22115, a self-supporting sheet-like material of cross-linked fibrin material canbe used as a bio-mechanical barrier in the treatment of internal traumatic lesions, particularly for prevention of adhesion formation as a post-operative complication. The '115 Application discloses the mixing of a thrombin and calcium containing solution with a fibrinogen and Factor XIII containing solution. By using high thrombin concentrations to catalyze the conversion of fibrinogen into fibrin, the resulting fibrin material was found to be sufficiently rigid to be self-supporting and to have sufficiently small pore size to prevent the ingress of fibroblasts which causes the formation of adhesions. The resulting fibrin material, however, did not readily retain water. In fact water could be easily expelled from the fibrin material by compressing the material by hand. Thus, this classic type fibrin material could not be used to deliver drugs to a wound site while being reabsorbed into the body during the fibrinolytic process.

This invention overcomes these and other shortcomings in the prior art devices. Hydrogel fibrin has a tight structure constituted of thin fibers defined by a low pore size. Water is trapped in the "void volume" of the structure. The "void volume" is small, regular, and homogenously distributed through the entire film material. Water cannot leave the film structure, due to its internal energy, and is released from the fibrin structure depending on the fibrinolytic rate of the biopolymer. The release of a drug incorporated into the water or buffer is regulated by passive diffusion and, depending upon the molecular weight, solubility and the fibrinolytic process.

The removal of calcium from the process of forming a fibrin structure yields no lateral associations of protofibrils. The lack of associations of protofibrils corresponds to a high number of thin fibers per unit of volume, thus conferring a tight pore size in the fibrin structure. This tight pore size allows for water to remain trapped in the "void volume."

## Summary of the Invention

Other advantages and aspects of the present invention will become apparent upon reading the following description of the drawings and detailed description of the invention.

The present invention provides a method for producing a fibrin hydrogel material, the method comprising the steps of:

> providing a fibrinogen reagent;
> providing a phosphate buffer calcium chelating agent;
> providing thrombin essentially free of calcium; and
> mixing the fibrinogen, the phosphate buffer chelating agent, and thrombin to form the fibrin hydrogel material, wherein the fibrin hydrogel material comprises a fibrin material having a water content of at least 90% by weight of the material and whereby the material retains at least 80% of the water upon compression by a force of 156G.

The present invention also provides an ex-vivo method for delivering fibrin hydrogel to a surface comprising the steps of:

> providing a liquid solution of fibrinogen;
> providing a liquid solution of thrombin essentially free of calcium;
> providing a liquid solution of a phosphate buffer calcium chelating agent;
> providing a spray unit in fluid communication with the fibrinogen solution, the thrombin solution, and the phosphate buffer chelating agent, the spray unit being capable of atomizing the fibrinogen solution, the thrombin solution, and the phosphate buffer chelating agent into an aerosol with at least one energy source of a liquid energy, a mechanical energy, a vibration energy, and an electric energy;
> spraying the fibrinogen solution onto the surface with the spray unit;
> spraying the phosphate buffer chelating agent onto the surface with the spray unit;
> spraying the thrombin solution onto the surface with the spray unit; and,
> mixing the fibrinogen solution, the phosphate buffer chelating agent and the thrombin solution,
> wherein the fibrin hydrogel comprises a fibrin material having a water content of at least 90% by weight of the material and whereby the material retains at least 80% of the water upon compression by a force of 156G.

There is also disclosed a medical device obtainable by said method for the prevention of post-surgical adhesion formation and the controlled release of drugs inhuman and non-human species. The device comprises a fibrin hydrogel material having a water content of at least about 90% by weight of the hydrogel. The fibrin hydrogel has a pore size within the range of less than 1 micron and preferably less than 0.1 $\mu$m has a transparency of less than about 1.0 AUFS, more preferably less than about 0.8 AUFS when measured with a spectrophotometer at 800 nm. The fibrin hydrogel is substantially free of cross-linking.

## Detailed Description of the Invention

While this invention is susceptible of embodiment in many different forms, there is shown in the drawings and will herein be described in detail a preferred embodiment of the invention with the understanding that the present disclosure is to be considered as an exemplification of the principles of the invention and is not intended to limit the broad aspect of the invention to the embodiments illustrated.

One preferred form of the present disclosure provides, a self-supporting, biodegradable, fibrin hydrogel material which is obtained by mixing fibrinogen and thrombin solutions diluted with a solute inhibiting the action of calcium on fibrinogen in a high ionic strength medium, both free of calcium. The prior art discloses that calcium is a critical component to forming a fibrin material. The resulting hydrogel material has thin fibers and small pore size and is suitable for use as an anti-adhesion barrier.

## I. Fibrin Hydrogel Material

Preferably the fibrin hydrogel material for anti-adhesion applications willhave a pore size from 1-5 microns, and more preferably from 0.1- 3. The fibrin hydrogel material preferably also readily retains water upon compression. Preferably the hydrogel shall retain 80 - 90 % of its water content upon compressing the material with a force from 1-14 psi.

The hydrogel material has a sufficiently high modulus of elasticity to be self-supporting. By self-supporting we mean that a fibrin hydrogel material of 5 cm long by 5 cm wide by 5mm thick can be held at one end without the second end deflecting downward with respect to the held end more than 10 degrees.

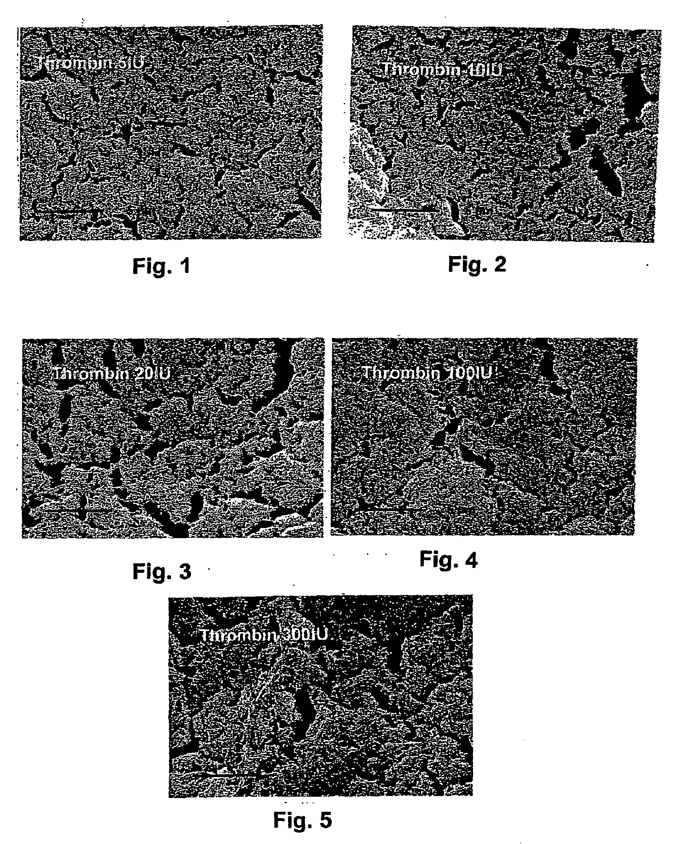

It is also desirable that the fibrin hydrogel be relatively optically transparent and, in a preferred form, should have an optical density measured with a spectrophotometer at 800 nm of from 0.1-0.2, more preferably from 0.1-0.5 and most preferably from 0.1-0.4. As can be seen in Figure 1, the fibrin hydrogel has a network of fibers 10 that should have an average diameter, in a preferred form, of less than about 5.0 microns, more preferably from less than about 2.0 microns and most preferably from less than about 1.0 microns or any range or combination of ranges therein.

The fibrin hydrogel is a single layered material.

In preferred embodiments, the thickness of the fibrin barrier material is at least 200$\mu$m when the barrier is in the wet state. Preferably the thickness is about 50$\mu$m, and most preferably up to 10,000$\mu$m, although it is believed that even material with a thickness of less than 100$\mu$m may be suitable for the disclosed purposes.

The hydrogel material must also be capable of being reabsorbed into the body or be bioreabsorbable. Preferably, depending upon the concentration of fibrinogen and the quantity applied, a fibrin hydrogel of 3 cm x 3cm x 1 cm the hydrogel will be reabsorbed into the body in its entirety by 14 days, more preferably within 10 days and most preferably within 5 days.

The fibrin hydrogel can be distinguished from classic fibrin material in several ways. First, the fibrin hydrogel can obtain a lower pore size than that of classic fibrin material with the same concentration of thrombin. The fibrin hydrogel has a tighter pore size regardless of the concentration of thrombin used. This has an advantage over classic fibrin materials in that the fibrinolytic process is maintained or increased over physiological levels.

Classic fibrin materials utilize large quantities of calcium, thus hampering the fibrinolytic process. The fibrinolytic process is slowed by the presence of gamma-gamma cross-links in the fibrin material, which are caused by the presence of excess calcium. The lower level of calcium in the fibrin hydrogel material inhibits cross-linking, thus allowing for a faster breakdown of the fibrin hydrogel material.

The increased time required by classic fibrin materials to be broken down may also result in a greater degree of adhesion. The anti-adhesion qualities of the fibrin hydrogel are believed to result from the thrombin content. The thrombin content of the fibrin hydrogel allows for a higher fibrinolytic rate than classic fibrin materials.

Another distinction is the fibrin hydrogel's ability to retain water under compression forces. The degree of water retention in the fibrin hydrogel greatly exceeds the water retention of the classic fibrin materials. This permeability factor is a primary distinction between the fibrin hydrogel of the present invention and classic fibrin materials. The slow rate of water release by the fibrin hydrogel material allows the hydrogel to act as a lubricant by release of water, further enhancing its anti-adhesion properties.

## II. Method of Forming a Fibrin Hydrogel

It has been found by the present inventors that a fibrin hydrogel material may be formed in the absence of a calcium containing solution and in the absence of a Factor XIII containing solution which were previously considered in the art to be essential components. In a preferred form of the invention a fibrin hydrogel is obtained by admixing a fibrinogen-containing solution with a thrombin-containing solution. The fibrinogen solution should have from 1.5-100 mg/ml, more preferably 3-70 mg/ml and most preferably a 45 mg/ml fibrinogen dissolved in a solution containing components capable of chelating calcium. The chelating component should also be non-toxic and in a preferred form of the invention is a phosphate buffer saline solution (PBS) of physiologically acceptable levels. The chelating agent should be an antagonist to fibrinopeptide transmidation reaction 5 IU-300 IU.

Also, contrary to the teachings in the prior art, the thrombin concentration of the admixed components does not determine the pore size of the hydrogel fibrin material. As will be discussed below and as shown in Figures 1-5, fibrin hydrogel materials were formed having relatively the same pore size notwithstanding the use of thrombin concentrations from 1 IU to 300 IU. The concentration of thrombin was still found to control the rate of forming a fibrin hydrogel.

It is well known that mixing a first solution containing fibrinogen with Factor XIII and a second solution of thrombin with calcium will result in the formation of a fibrin material with pronounced lateral association and considerable cross-linking among its thick fibers. It is also well known that thrombin acts as a protease which will cleave fibrinopeptide A and B from the fibrinogen molecule and convert it into fibrin. The fibrinopeptides of vertebrate species reportedly have a large net negative charge. The presence of these and other negatively charged groups in the fibrinopeptides are likely factors in keeping fibrinogen apart. Their release by thrombin gives fibrin monomers a different surface-charge pattern, leading to their specific aggregation. In particular, removal of the fibrinopeptides changes the net charge of the central globular unit from -8 to +5. Each of the terminal globular units has a net charge of -4. Thus, electrostatic interactions between the terminal and central globular units probably stabilize the structure of fibrin.

It is also known that calcium ions play an important role in the dissociation of Factor XIII subunit A from Factor XIII subunit B as Factor XIII is converted to its activated form, Factor XIIIa. Furthermore, it is know that Factor XIIIa is critical to the cross-linking of fibrin monomers. It is also known that the widths ofthe fibers comprising the fibrin material can be decreased by increasing the pH and ionic strength of the diluents.

By removing (chelating) calcium ions bound to fibrinogen, the inventors have been able to modify the fibrin structure to obtain further embodiments of the fibrin hydrogel. Accordingly, the present invention uses a solution capable of scavenging calcium ions associated with the fibrinogen molecules. In a preferred form of the invention, the solution is a phosphate buffer solution having a concentration similar to physiologically acceptable levels. The inventors suggest that the resulting structural modifications of the fibrin hydrogel occur as a result of a "charge effect" which alters the aforementioned electrostatic interactions between the terminal and central globular units, thereby inhibiting the lateral association of fibrin. In further embodiments of the fibrin hydrogel, modification of Factor XIII concentration used in the synthesis of fibrin alters the crosslinking characteristics of the final fibrin material. Thus, in further embodiments of the invention, the inventors have developed a fibrin hydrogel that can be synthesized with low concentrations of thrombin and according to the end user's specifications as to the lateral association and fiber thickness of the resulting fibrin hydrogel structure.

During the formation of a fibrin hydrogel, it is desirable that all of the fibrinogen be converted into fibrin, as residual amounts of fibrinogen may lead to adhesion formation upon reacting with thrombin present in the body. Accordingly, in still further embodiments of the present invention, the fibrin hydrogel further comprises less than 5% by weight of fibrinogen, preferably less than 4% by weight of fibrinogen, preferably less than 3% by weight of fibrinogen, preferably less than 2% by weight of fibrinogen, and most preferably less than 1% by weight of fibrinogen, in terms of the total dry weight of the fibrinogen plus fibrin each time. For the purpose of determining the fibrin and the fibrinogen content of the fibrin film, the methods of SDS-Page (SDS-Gel Electro-phoresis) may be used.

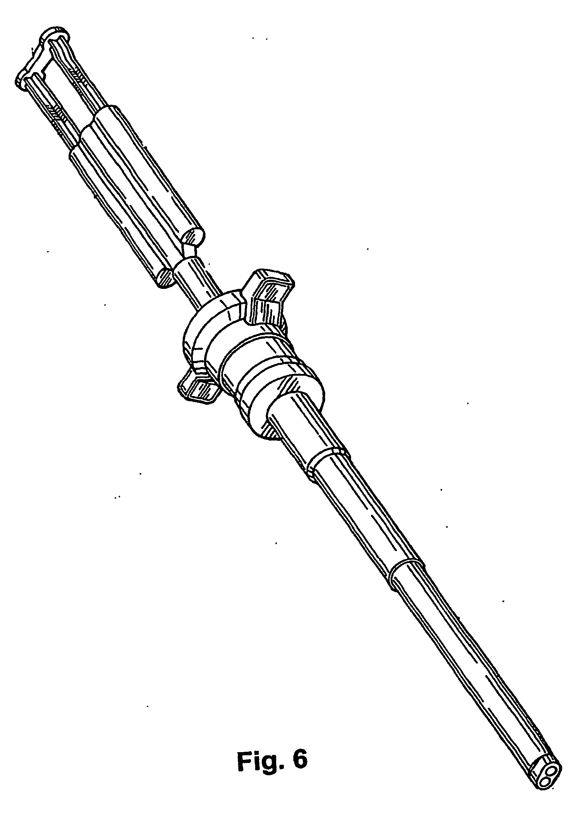

The medical devices shown in Figure 6 and as further described in commonly assigned U.S. Patent No. 5,989,215 may be used topically, in open-type surgeries (for example, laparotomic surgeries) or minimally invasive surgeries (for example, laparoscopic surgeries). Of course, there are other types of open-type surgeries and minimally invasive surgeries as will be appreciated by one of ordinary skill in the art. The medical device 20 may be used to form fibrin hydrogel material inside and outside the animal body.

There is also disclosed a process for preparing a self-supporting fibrin hydrogel matrix or film outside the body comprising the steps of:

(a) mixing a stream of a first, fibrinogen-containing solution dissolved in PBS with a stream of a second, thrombin-containing PBS solution;
(b) applying the obtained mixture to a solid support or mixing the components of the solid support; and
(c) incubating the mixture to form the hydrogel matrix.

In order to obtain a mixture as homogenous as possible (and thus a homogenous final product) in step (a) a stream of a first, fibrinogen-containing solution is mixed with a stream of a second, thrombin-containing solution by simultaneous delivery of the components. It is also possible to deliver one component to a surface followed by the other component. Preferably, equal volumes of the first and the second solution are mixed. In case the different volumes of the first and the second solution should be mixed, it will be known in the art which measures have to be taken in order to ensure that a homogenous mixture is obtained.

Using the delivery device mentioned above, the resulting mixture is spread over the surface of a solid support, for example a petri dish or the like, which is tilted to cover the entire surface as far as possible before the formation of the fibrin hydrogel material begins.

For the purpose of preparing a fibrin hydrogel on mammal tissue, the inventors propose a process comprising the steps of:

(a) providing a first phosphate buffer solution containing fibrinogen;
(b) providing a second phosphate buffer solution containing thrombin;
(c) mixing the first solution and the second solution before or after placing the mixture on an animal tissue;
(d) and obtaining a fibrin hydrogel material with a tight structure and small pore size suitable for post-surgical adhesion prevention.

The fibrinogen and thrombin solutions can be initially mixed in a delivery device, or be atomized into a spray and mixed while in the form of spray droplets while in mid air or upon first making contact with the tissue surface or delivered through a multi-lumen catheter.

The fibrin hydrogel may be formed by utilizing constituents of a kit. A preferred embodiment of the fibrin hydrogel kit includes:

(a) a vial of proteins including fibrinogen;
(b) a vial of thrombin;
(c) a vial of phosphate buffer solution to serve as a diluent; and
(d) appropriate ancillary mixing and application apparatus, including, but not limited to syringes and catheters.

One further embodiment of the fibrin hydrogel kit includes a vial containing the protein cocktail where the protein content is no less than 30 mg/ml. Another further embodiment of the fibrin hydrogel kit includes a vial containing the protein cocktail where the Factor XIII content ranges from 0 IU/ml to 80 IU/ml. Yet a further embodiment of the fibrin hydrogel kit includes a vial containing thrombin, where the concentration of thrombin ranges between 0.1 IU/ml to 1000 IU/ml. In yet another preferred embodiment of the fibrin hydrogel kit, the constituents supplied are pre-formulated to ensure that when mixed, the hydrogel achieved will have a homogeneous structure with tight pore sizes suitable to act as a prophylaxis to adhesion formation.

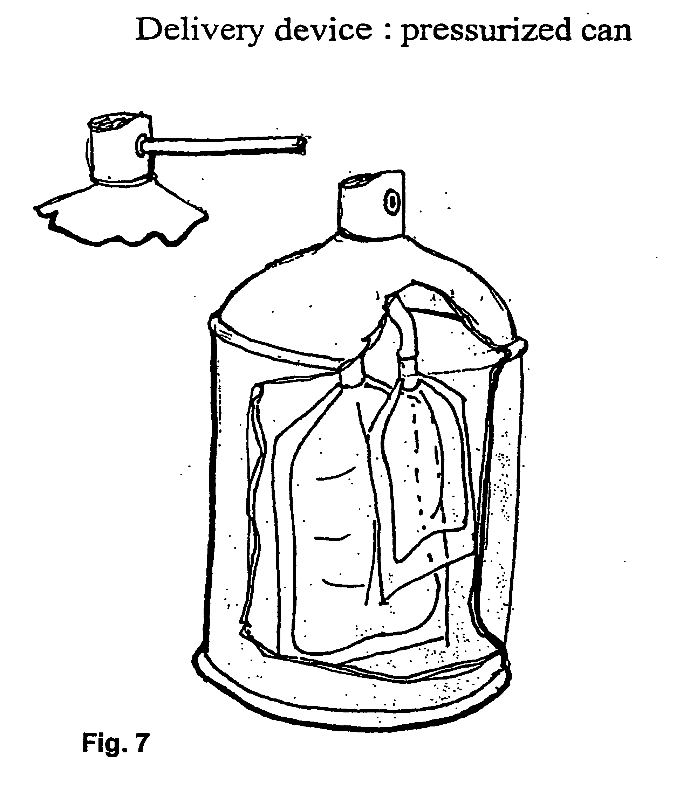

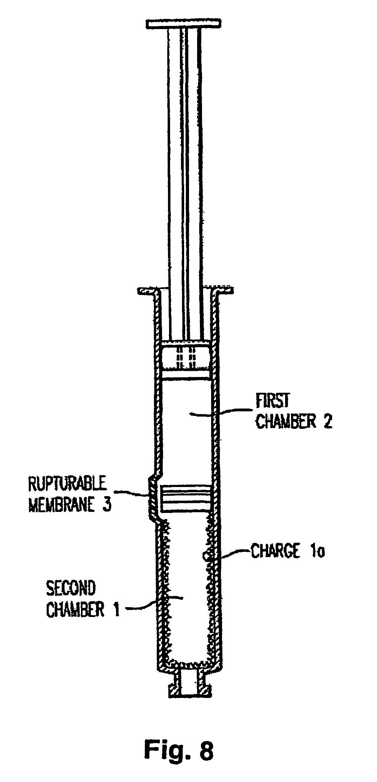

Another preferred embodiment of the fibrin hydrogel kit includes pre-formulated constituents, supplied to ensure that when mixed, the hydrogel achieved will have a homogeneous structure with tight pore sizes suitable to act as a prophylaxis to adhesion formation and as a drug delivery system. A lack of adhesion can be seen with in fibrinogen free of FXIII or similar acting component The fibrin hydrogel kit includes a vial of fibrinogen mixed with inactivated thrombin, a vial of suitable buffer, an ancillary, a device equipped with optical fiber to photoactivate the thrombin, and a light supply. In another preferred embodiment of the fibrin hydrogel kit, the fibrinogen, inactivated thrombin, suitable buffer, and an ancillary are all in one delivery device. Figure 7 illustrates another preferred embodiment where a pressurized canister houses fibrinogen and thrombin as powder in separate bags. The canister may be unscrewed to allow for rehydration of the fibrinogen and thrombin. Increasing the pressure allows both components to be sprayed through a double tube system or through concentric channels. Another preferred embodiment, Figure 8, utilizes a double syringe system with volumes of less than 100, 50, 20 ml each, more preferably less than 20 ml each, and most preferably less than 10 ml each, but greater than 3.0 ml. This double syringe system is equipped with a Y-shaped connection to incorporate a tube. Such devices can be used for veterinary applications. In another preferred embodiment, the fibrin hydrogel material can be fabricated into articles selected from the group consisting of films, tubes, and pellets. These fibrin hydrogel materials can be fabricated into articles using techniques selected from the group of extrusion, molding, and thermal forming. These fibrin hydrogel materials can be sterilized at a temperature below 0 °C by gamma radiation, stored at a temperature below 0°C, and used upon demand. The sterilization by gamma radiation is below - 25 ° C, at a dosage of at least 25 kGy. In yet another preferred embodiment of the fibrin hydrogel kit, the vial of diluent contains a phosphate buffer solution. In another preferred embodiment of the fibrin hydrogel kit, the vial of diluent contains a high-ionic strength buffer capable of scavenging the fibrinogen-linked calcium. Other suitable solutions include; sodium citrate, potassium citrate, EDTA, EGTA, chloride solutions, phosphate solutions, or other ions solutions having a strong affinity for calcium. Fibrinogen, thrombin, and other proteins such as fibronectin and FXIII maybe from a single donor, multiple donors, pooled donors, Cohn I fraction, or recombinant. In another preferred embodiment of the fibrin hydrogel kit, the vial of diluent contains a buffer capable of chelating exogenous calcium.

### III. Therapeutic Fibrin Hydrogel

There is disclosed a fibrin hydrogel that releasably retains a diluent or a therapeutic agent. The therapeutic agent is retained within the pores of the hydrogel material and when placed into the body of a mammal is released over time as the fibrin hydrogel is reabsorbed into the body.

The therapeutic agent(s) that are contemplated to be releaseably retained by the therapeutic hydrogel layer comprises, but is not limited to, pharmaceutical compounds, antibiotics, fibrinolytic agents, and biological response modifiers, in particular cytokidnes and wound repair promoters, preferably in an amount up to 1% by weight in terms of the total dry weight of fibrin plus fibrinogen. Due to the chemotactive properties of thrombin, low thrombin concentration is preferred for the purpose of anti-adhesion. However, higher concentrations of thrombin may be required to hasten clotting time. Clotting time was performed with a semi-automated BFT II device from Dade Behring on fibrinogen at 25 mg/ml with varying thrombin concentrations of 0.5,1.0,2.5,5.0, and 10.0 IU/ml. PBS was used as a diluent for fibrinogen and thrombin. The clotting times are listed in the table below.

| Fibrinogen Concentration | Thrombin Concentration | Clotting Time (Seconds) |
| --- | --- | --- |
| 25 mg/ml | 0.5 IU/ml | 430 |
| 25 mg/ml | 1.0 IU/ml | 237 |
| 25 mg/ml | 2.5 IU/ml | 83 |
| 25 mg/ml | 5.0 IU/ml | 37 |
| 25 mg/ml | 10.0 IU/ml | 20 |

Examples of fibrinolytic agents include t-PA, $\mu$-PA, streptokinase, staphylokinase, plasminogen and the like. These compounds promote fibrinolysis and thus can be used for controlling the rate of the degradation of the fibrin film in vivo. The term *"biological response modifiers"* is meant to refer to substances which are involved in modifying a biological response, such as wound repair, in a manner which enhances the desired therapeutic effect Examples include cytoldnes, growth factors, and the like. Due to its intrinsic mechanical properties, the fibrin film of the invention does not require any additional cross-linking agent which may exert any toxical effects to the human or animal body. Due to its high level of dilution, it is possible for the fibrin hydrogel to trap and release water. This is useful for the hydration of tissues or as a lubricant to assist in the anti-adhesive properties of the fibrin hydrogel.

The therapeutic agent can be incorporated into the fibrin hydrogel material during the formation of the hydrogel. The therapeutic agent may eitherwater soluble or water insoluble, antibody, antimicrobial agent, agent for improving biocompatability, proteins, antiinflammatory compounds, compounds reducing graft rejection, living cells, cell growth inhibitors, agent stimulating endothelial cells, antibiotics, antiseptics, analgesics, antineoplastics, polypeptides, protease inhibitors, vitamins, cytokines, cytotoxins, minerals, interferons, hormones, polysacharides, genetic material, growth factors, cell growth factors, substances against cholesterol, pain killers, collagens, stromal cells, osteo-progenitor cells, polylactate, alginate, $C_2$-$C_{24}$ fatty acids, and mixtures thereof. The delivery of the therapeutic agent regulated by either or both the passive diffusion and the fibrinolytic rate. The therapeutic agent can be dissolved in one or both of the thrombin or fibrinogen solutions. The therapeutic agent is retained by the hydrogel material as it forms out of the admixed solution.

It is also preferred that the fibrinogen solution have a viscosity that allows the solution to be sprayed and preferably sprayed using pressures generated using a hand-operated syringe. The fibrinogen solution should have a viscosity of less than 20 centipoise, more preferably less than 10 centipoise, and most preferably from 1-5 centipoise or any combination or subcombination of ranges therein. The thrombin-containing solution should have a thrombin concentration less than 10000 IU thrombin. The fibrin glue has been preferably made by mixing said fibrinogen-containing solution with an equal volume of a thrombin-containing solution of at least 50 IU thrombin, preferably of at least 150 IU thrombin, and most preferably of at least 300 IU thrombin

Other contemplated embodiments include:

1. Beads of hydrogel material between 0.1mm and 3mm.
2. A hydrogel material anatomically molded.

There is disclosed a fibrin hydrogel that retains a higher proportion of water than fibrin materials currently available. The greater degree of water retention is particularly beneficial to the therapeutic use of the hydrogel. The retention of water is necessary for the control of the concentration of therapeutic agents contained within the fibrin hydrogel, as well as for the effective release of these therapeutic agents and additives.

The ability of fibrin hydrogels to retain water while being subjected to compression forces was tested and compared to the water retaining capacity of a classic fibrin material. In particular, compression was applied by centrifugation of the materials at various rotational speeds and the amount of water retained was measured. A refrigerated centrifuge (Sorvall RT 6000B) spun fibrin hydrogels at different speeds:

- 1000 rpms for 30 min. corresponding to 156G
- 2000 rpms for 30 min. corresponding to 625G
- 3000 rpms for 30 min. corresponding to 1428G

Amicon filter type "centricon 30" was used, corresponding to a membrane cutoff of 30000 and characterized by a maximum rotation time of 30 min and sustaining a G-force max of 5000G. The Amicron filter is composed of two units. The upper unit contains the filter component itself and can be attached to the second unit of the Amicron filter. The second, or lower, unit is the bottom cup. The bottom cup allows for the collection of water that is expelled from the fibrin material deposited on the filter of the upper unit. The water collected in the bottom cup is used to measure the amount of water released by the fibrin materials at the various rotational speeds. Once fibrinogen and thrombin solutions were prepared, a volume of approximately 1 ml of fibrin was applied to the filter. An appropriate mixing device is required for the fibrinogen and thrombin mixture to be complete and homogeneous.

Upper and lower parts are separately weighed before the fibrin material deposition and after each centrifugation step. A correction factor is calculated in order to consider that 1 g of fibrin has been distributed on the filter.

Separate experiments were conducted to test the effects of diluents. The procedural steps for each experiment went as follows:

1) The filter and the bottom cup of the Amicon filter are separately weighed,
then the fibrin material obtained by mixing each fibrinogen solution with a 20IU/mL thrombin solution is put on the filter.
A correction factor is calculated in order to consider that 1 g of fibrin has been distributed on the filter.
The filter is centrifuged at 1000 rpm for 30 minutes.

At the end of the centrifugation cycle, the bottom cup is carefully removed, weighed and recorded.

2) The bottom cup is connected to the filter and centrifuged at 2000 rpm for 30 minutes, at the end of the cycle, the bottom cup is weighed and the cumulative value recorded.

3) Again the bottom cup is connected to the filter and centrifuged at 3000 rpm for 30 minutes and the bottom cup weighed at the end of the cycle.

In the first experiment, the fibrinogen vial was reconstituted with 3.5 ml- distilled water to obtain a final concentration of 100 mg/ml of fibrinogen. Dilutions of the fibrinogen were performed with water in order to respectively obtain:

- dilution 1:2 (50mg/ml) in water
- dilution 1:4 (25mg/ml) in water
- dilution 1:6 (16.6mg/ml) in water
- dilution 1:8 (12.5mg/ml) in water

Thrombin (Baxter Hyland) was reconstituted with 3.5 ml of 40mmol $CaCl_2$ in order to obtain a concentration of 300IU/mL. A dilution is performed with $CaCl_2$ to obtain a thrombin concentration of 20IU/mL.

Fibrinogen solutions were then mixed with an equal volume of thrombin (20IU/mL) to obtain a final concentration of "fibrinogen" respectively of:

- Sample 1: diluted 1:4 (25mg/ml)
- Sample 2: diluted 1:8 (12.5mg/ml)
- Sample 3: diluted 1:12 (8.3mg/ml)
- Sample 4: diluted 1:16 (6.25mg/ml)

For sample 1, the loss of water was 9.5%, 25%, and 45% at 1000, 2000, and 3000 rpm respectively. Sample 2 showed a loss of water of 18%, 40%, and 70% at 1000, 2000, and 3000 rpm respectively. The loss of water in sample 3 was 20%, 40%, and 80% at 1000, 2000, and 3000 rpm respectively. Sample 4 expressed a water loss of 20%, 72%, and 90% at 1000, 2000, and 3000 rpm respectively. See Table 1 below.

**Table 1**
**Fibrinogen & Thrombin in Water**

The second experiment was a comparison of water retention between fibrin obtained by mixing fibrinogen and thrombin respectively reconstituted and diluted in PBS and fibrinogen reconstituted and diluted in PBS with thrombin reconstituted and diluted in 40mmol calcium chloride.

In these comparisons, a vial of fibrinogen was reconstituted with 3.5 ml PBS (phosphate buffered saline pH=7.2) to reach a final concentration of 100mg/mL of fibrinogen.

Dilutions were performed from this vial in order to obtain fibrinogen concentrations of:

- Sample 11:2 (50mg/ml) in PBS
- Sample 2 1:4 (25mg/ml) in PBS
- Sample 31:6 (16.6mg/ml) in PBS
- Sample 41:8 (12.5mg/ml) in PBS

In experiment 2 A, thrombin (Baxter Hyland) was reconstituted with 3.5 mL ofPBS in order to obtain a concentration of 300IU/mL. A dilution was performed with PBS to obtain a thrombin concentration of 20IU/mL.

In experiment B, thrombin (Baxter Hyland) was reconstituted with 3.5 ml of 40mmol CaCl2 (calcium chloride) in order to obtain a concentration of 300IU/mL. A dilution was performed with cacl2 to obtain a thrombin concentration of 20IU/mL.

The fibrinogen solutions were then mixed with an equal volume of thrombin (20IU/mL), resulting in final concentrations for fibrinogen of:

- Sample 1: diluted 1:4 (25mg/ml) in PBS
- Sample 2: diluted 1:8 (12.5mg/ml) in PBS
- Sample 3: diluted 1:12 (8.3mg/ml) in PBS
- Sample 4: diluted 1:16 (6.25mg/ml) in PBS

For experiment 2 A, sample 1 expressed a loss of water that was 6%, 10%, and 14% at 1000, 2000, and 3000 rpm respectively. Sample 2 showed a loss of water of 10%, 21 %, and 35% at 1000, 2000, and 3000 rpm respectively. The loss of water in samples 3 and 4 was nearly identical at 11%, 20%, and 35% at 1000, 2000, and 3000 rpm respectively. See Table 2A below.

**Table 2A**
**PBS Diluent for Fibrinogen & Thrombin**

The introduction of calcium to the fibrin formulation through the diluent used for the thrombin dilutions in experiment 2 B directly affected the water retention. The loss of water was not significant at 1000 rpm for the samples, but water losses increased significantly to approximately 40% at 2000 rpm for samples 2 through 4. At 3000 rpm, water loss increased to approximately 65% for samples 2 and 3. A water loss of 80% for sample 4, similar to the result obtained for fibrin described in experiment 1, was recorded at 3000 rpm. The results for these experiments support the hypothesis that fibrin structures essentially free of calcium ions are also tighter, more compact, and have a greater resistance to water loss from compression forces. See Table 2B below.

**Table 2B: PBS Diluent for Fibrinogen**
**CaCl2 for Thrombin (20IU)**

As a means of verification for the role of phosphate as a complexing agent of the remaining calcium ions on fibrinogen molecules, reproductions of the PBS experiment above were conducted substituting EDTA for PBS in one

trial, and citrate of potassium for PBS in a second trial. The patterns of water loss for both the EDTA and citrate of potassium trials were consistent with the results from the experiment utilizing PBS as a reconstitution agent. See Tables 3A and 3B below. These results sustain the hypothesis that remaining calcium on fibrinogen reacts with phosphate ions preventing the collateral association of protofibrils producing a tight fibrin structure more resistant to water loss than fibrin structures retaining calcium.

**Table 3A**
**Fibrinogen (EDTA 50 mM)**

**Table 3B: K Citrate as Diluent for Fibrinogen and Thrombin**

Another experiment was conducted to determine the impact of the FXIII present in the formulation on the compaction capability of the fibrin material obtained with PBS as diluent for both fibrinogen and thrombin. In this experiment, a vial of fibrinogen (Tisseel from Baxter Hyland-Immuno lot P5488797D) is reconstituted with 4.0 ml of PBS (dilution 1:2) to reach a final concentration of 50mg/mL of fibrinogen.

Dilutions are performed from this vial in order to obtain a fibrinogen concentration respectively of:

- Dilution 1:2 (50mg/ml) in PBS
- Dilution 1:4 (25mg/ml) in PBS
- Dilution 1:6 (16.6mg/ml) in PBS
- Dilution 1:8 (12.5mg/ml) in PBS

Thrombin (Baxter Hyland) is reconstituted with 3.5 ml of PBS in order to obtain a concentration of 300IU/mL. A dilution is performed with PBS to obtain a thrombin concentration of 20IU/mL. The results of this experiment show that there is no difference between the Baxter Fibrin sealant containing FXIII (experiment 2) and the Baxter Hyland-Immuno free of FXIII when submitted to the compaction test. Results obtained from the compaction tests show the same behavior for the FXIII free sealant. See Table 4 below.

**Table 4:  PBS as Diluent for Fibrinogen (no FXIII) & Thrombin 20IU**

A table summarizing the water retention data follows below.

Table 5

| Sample # | Experiment # | % Water loss at 1000 rpm | % Water loss at 2000 rpm | % Water loss at 3000 rpm |
|---|---|---|---|---|
| 1 | 1 | 9.5 | 25 | 45 |
| 1 | 2A | 6 | 10 | 14 |
| 1 | 2B | not significant | 40 | 65 |
| 1 | 4 | 4.2 | 9.4 | 14.3 |
| 2 | 1 | 18 | 40 | 70 |
| 2 | 2A | 10 | 21 | 35 |
| 2 | 2B | not significant | 40 | 65 |
| 2 | 4 | not significant | 15 | 22 |
| 3 | 1 | 20 | 40 | 80 |
| 3 | 2A | 11 | 20 | 35 |
| 3 | 2B | not significant | 40 | 65 |
| 3 | 4 | not significant | 23 | 34 |
| 4 | 1 | 20 | 72 | 90 |
| 4 | 2A | 11 | 20 | 35 |
| 4 | 2B | not significant | - | 80 |
| 4 | 4 | 14 | 24 | 34 |

It has been postulated that ionic strength of thrombin regulates the pore size of fibrin clot structure. By using high ionic strength thrombin solutions one can achieve a fibrin clot having a smaller pore size than with lower concentration thrombin solutions. The present example demonstrates a method for fabricating a fibrin clot material where the concentration does not govern the pore size of the fibrin clot. As set forth above, by using a chelating agent to bind to calcium, calcium concentration does not affect the pore size. Even when a 4 IU/ml thrombin concentration and a 250 IU/ml thrombin concentration was used, the resulting fibrin material had substantially the same pore size. Ionic strength was measured with an osmometer and correlated with the measurement of the turbidity at 800 nm with a spectrophotometer. Observation of the sample network structure was accomplished by scanning electron microscopy. Table 6 below summarizes the correlation between final osmolarity of the fibrin samples and their respective optical densities. The results of this experiment illustrate that ionic strength, as demonstrated through osmolarity, does not regulate the structure of the fibrin clot.

Classic fibrin materials obtained by using water (0 mosm) as a diluent for fibrinogen and $CaCl_2$ for thrombin (4

IU/ml), for example, has a final osmolarity of 539 mosm. This is a result of fibrinogen reconstituted with water at a concentration of 90 mg/ml (610 mosm) combined with thrombin reconstituted with $CaCl_2$ at a kit concentration of 4 IU/ml (468 mosm). The resulting classic fibrin material is opaque white with an optical density of 2.8 AUFS when measured with a spectrophotometer at 800 nm.

Fibrin materials produced with PBS (286 mosm) have a final osmolarity of 445 mosm. This is a result of fibrinogen reconstituted and diluted with PBS at a concentration of 25 mg/ml (588 mosm) combined with thrombin reconstituted and diluted with PBS at a concentration of 10 IU/ml (315 mosm). The resulting fibrin material is optically clear with an optical density of 0.5 AUFS when measured with a spectrophotometer at 800 nm.

Fibrin materials produced with a citrate buffer at 0.033 M (100 mosm) have a final osmolarity of 224 mosm. This is a result of fibrinogen reconstituted and diluted with citrate at a concentration of 50 mg/ml (336 mosm) combined with thrombin reconstituted and diluted with citrate at a concentration of 20 IU/ml (112 mosm). The resulting fibrin material is optically clear with an optical density of 0.45 AUFS when measured with a spectrophotometer at 800 nm. The fibrin hydogel material in this experiment is clear and composed of thin fibers with an ionic strength of less than 300 mosm, which is considered to be the physiological level. Fibrin obtained by mixing fibrinogen at 12.5 mg/ml with thrombin at 10 IU remains clear (0.8 AUFS) with an osmolarity of 167 mosm.

Fibrin materials produced with a citrate buffer at 0.066 M (190 mosm) have a final osmolarity of 317 mosm. This is a result of fibrinogen reconstituted and diluted with citrate at a concentration of 50 mg/ml (435 mosm) combined with thrombin reconstituted and diluted with citrate at a concentration of 20 IU/ml (200 mosm). The resulting fibrin material is optically clear with an optical density of 0.23 AUFS when measured with a spectrophotometer at 800 nm. The fibrin hydogel material in this experiment is also clear and composed of thin fibers with an ionic strength of less than 300 mosm, which is considered to be the physiological level. Fibrin obtained by mixing fibrinogen at 12.5 mg/ml with thrombin at 10 IU remains clear (0.5 AUFS) with an osmolarity of 260 mosm.

Table 6

| Buffer | Final Osmolarity of Fibrin Material (mosm) | Optical Density (AUFS at 800 nm) |
|---|---|---|
| Water ($CaCl_2$) | 539 | 2.8 |
| PBS | 451 | 0.255 |
| Citrate (0.033 M) | 224 | 0.45 |
| Citrate (0.066 M) | 317 | 0.23 |

The buffers also play an active role in the permeability, fiber diameter, and mass length ratio of the fibrin material. Significant differences can be observed between PBS and NaCl (0.15 M). These buffers have the same osmolarity, yet their effects on fibrin materials are markedly different. See Table 7 below. For a thrombin concentration of 2 IU/ml reconstituted with NaCl at 0.15 M, the permeability of the fibrin is $30.6 \times 10^{-12}$ at a fibrinogen concentration of 25 mg/ml and $136 \times 10^{-12}$ at a fibrinogen concentration of 12.5 mg/ml. Using PBS as a buffer yields a fibrin permeability of $6.9 \times 10^{-12}$ at a fibrinogen concentration of 25 mg/ml and $39.5 \times 10^{-12}$ at a fibrinogen concentration of 12.5 mg/ml. This experiment demonstrates that the fibrin material utilizing PBS as a buffer is nearly five times less permeable than classic fibrin materials, thus capable of retaining more water. The diameters of fibers are also affected by the buffer selected. For a thrombin concentration of 2 IU/ml diluted with NaCl at 0.15 M, the fibers have a diameter of 0.107 $\mu$m at a fibrinogen concentration of 25 mg/ml and 0.14 $\mu$m at a fibrinogen concentration of 12.5 mg/ml. Using PBS as a buffer yields fibers with a diameter of 0.051 $\mu$m at a fibrinogen concentration of 25 mg/ml and 0.075 $\mu$m at a fibrinogen concentration of 12.5 mg/ml. Additionally, the mass length ratio is also affected by the buffer selected to reconstitute the fibrinogen and thrombin. At a thrombin concentration of 2 IU/ml buffered with NaCl at 0.15 M, the mass length ratio is $8.1 \times 10^{12}$ at a fibrinogen concentration of 25 mg/ml and $13.9 \times 10^{12}$ at a fibrinogen concentration of 12.5 mg/ml. The use of PBS as a buffer yields fibers with a mass length ratio of $1.83 \times 10^{12}$ at a fibrinogen concentration of 25 mg/ml and $4.03 \times 10^{12}$ at a fibrinogen concentration of 12.5 mg/ml, a four-fold reduction under NaCl at 0.15 M.

For a thrombin concentration of 250 IU/ml (Table 8) diluted with NaCl at 0.15 M, the permeability of the fibrin is $14.24 \times 10^{-12}$ at a fibrinogen concentration of 25 mg/ml and $47.7 \times 10^{-12}$ at a fibrinogen concentration of 12.5 mg/ml. Using PBS as a buffer yields a fibrin permeability of $8.9 \times 10^{-12}$ at a fibrinogen concentration of 25 mg/ml and $41 \times 10^{-12}$ at a fibrinogen concentration of 12.5 mg/ml. This experiment demonstrates that the fibrin material utilizing PBS as a buffer is less permeable than classic fibrin materials, thus capable of retaining more water. The diameters of fibers are also affected by the buffer selected. For a thrombin concentration of 250 IU/ml diluted with NaCl at 0.15 M, the fibers have a diameter of 0.073 $\mu$m at a fibrinogen concentration of 25 mg/ml and 0.083 $\mu$m at a fibrinogen concentration of 12.5 mg/ml. Using PBS as a buffer yields fibers with a diameter of 0.057 $\mu$m at a fibrinogen concentration of 25 mg/ml and 0.077 $\mu$m at a fibrinogen concentration of 12.5 mg/ml. Additionally, the mass length ratio is also affected by the buffer selected to reconstitute the fibrinogen and thrombin. At a thrombin concentration of 250 IU/ml buffered with NaCl at 0.15 M, the mass length ratio is $3.78 \times 10^{12}$ at a fibrinogen concentration of 25 mg/ml and $4.83 \times 10^{12}$ at a fibrinogen concentration of 12.5 mg/ml. The use of PBS as a buffer yields fibers with a mass length ratio of $2.36 \times 10^{12}$ at a fibrinogen concentration of 25 mg/ml and $4.24 \times 10^{12}$ at a fibrinogen concentration of 12.5 mg/ml.

These experiments illustrate that the type of buffer used, differently effects the permeability factor as shown for NaCl and PBS (Tables 7 and 8). These experiments demonstrate that the concentration of thrombin has no effect on the permeability factor, fiber diameter, and mass length ratio as well when PBS, and not NaCl, is the buffer. PBS is an admixture composed of 0.13 M NaCl (800 mg/ L), KCL (20 mg/L), anhydrous $Na_2HPO_4$ (115 mg/L), and $KH_2PO_4$. Thus, PBS buffer contains NaCl at a molarity of nearly the NaCl 0.015 M buffer described in Tables 7 and 8. As the data shows, phosphate is therefore the complexing agent of the endogenous calcium. Tables summarizing these experiments are labeled Table 7 and Table 8 below.

Table 7 - Thrombin 2 IU/ml

| Buffer | Fibrinogen Concentration | Permeability $(K_s)$ | Fiber Diameter $\mu m$ | mass length ratio |
|---|---|---|---|---|
| NaCl 0.15 M | 25 mg/ml | $30.6 \times 10^{-12}$ | 0.107 | $8.1 \times 10^{12}$ |
| PBS | 25 mg/ml | $6.9 \times 10^{-12}$ | 0.051 | $1.83 \times 10^{12}$ |
| NaCl 0.15 M | 12.5 mg/ml | $136 \times 10^{-12}$ | 0.14 | $13.9 \times 10^{12}$ |
| PBS | 12.5 mg/ml | $39.5 \times 10^{-12}$ | 0.075 | $4.03 \times 10^{12}$ |

Table 8 - Thrombin 250 IU/ml

| Buffer | Fibrinogen Concentration | Permeability (Ks) | Fiber Diameter $\mu m$ | mass length ratio |
|---|---|---|---|---|
| NaCl 0.15 M | 25 mg/ml | $14.24 \times 10^{-12}$ | 0.073 | $3.78 \times 10^{12}$ |
| PBS | 25 mg/ml | $8.9 \times 10^{-12}$ | 0.057 | $2.36 \times 10^{12}$ |
| NaCl 0.15 M | 12.5 mg/ml | $47.7 \times 10^{-12}$ | 0.083 | $4.83 \times 10^{12}$ |
| PBS | 12.5 mg/ml | $41 \times 10^{-12}$ | 0.077 | $4.24 \times 10^{12}$ |

Calculation for permeability (Ks):

$$\underline{\frac{\text{Flow (ml/sec) x time to clot x viscosity } (10^2)}{\text{Pressure x Surface area of clot}}}$$

Calculation for fiber diameter:

$$D^2 = 44.1 \text{ x Ks x concentration of fibrinogen } (X^{1.3736})$$

Calculation for mass length ratio:

$$\mu = \prod \text{ x } D^2 \text{ x C/ 4X,}$$

where C=4.36 g/cm$^3$

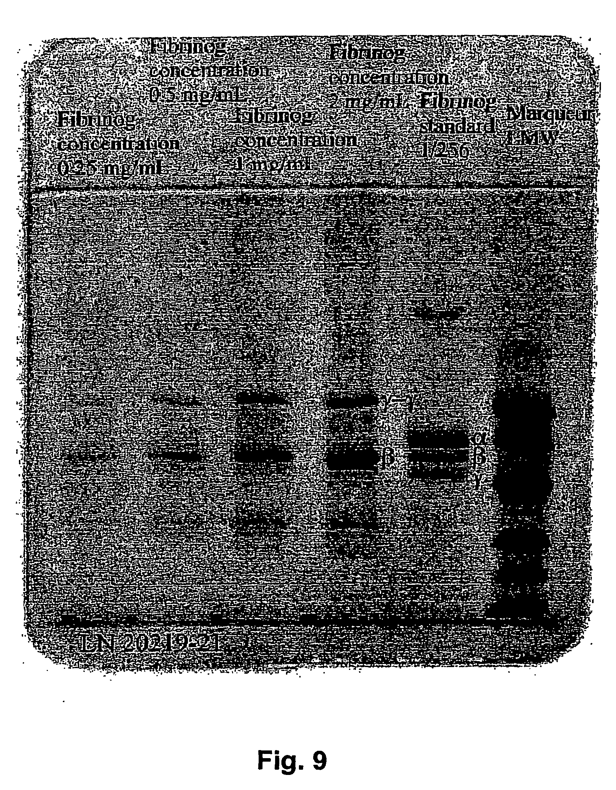

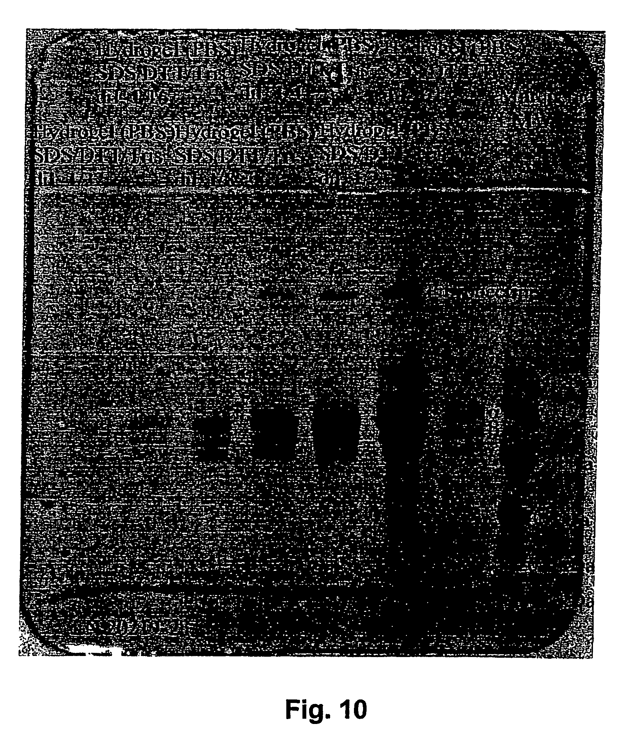

The role of PBS as a complexing agent can also be seen by gel electrophoresis studies of fibrin materials. When an electric current is applied to an SDS-polyacrylimide-gelelectrophoresis containing fibrin hydrogel material prepared with PBS, little or no gamma-gamma banding can be seen while a fibronectin band may be viewed. This demonstrates that the PBS complexes calcium so that calcium is not available to collaterally associate fibrin through cross-linking. Classic fibrin materials show distinctive, strong gamma-gamma band, and no fibronectin band, when prepared with water or NaCl as a buffer. NaCl buffer with a molarity of 0.15 cannot block the action of calcium. The NaCl buffer's inability to block the action of calcium allows calcium to play its traditional role in collaterally associating fibrin, thus allowing thrombin to affect the pore size of the fibrin material. By acting as a complexing agent for endogenous calcium, PBS substantially removes thrombin's ability to affect pore size. Figure 9 below illustrates the classic fibrin material in gel electrophoresis. Figure 10 below illustrates the fibrin hydrogel material in gel electrophoresis.

The fibrin hydrogel materials have also been determined to contain anti-adhesive properties. Tables 9 and 10 below illustrate the anti-adhesive properties of the fibrin hydrogel materials. Table 9 shows the results of a side model study on rats. The caecum and interfacing parietal wall were abraded sufficiently to cause bleeding. The bleeding surfaces were cauterized to stop the bleeding on the injured surfaces in both control and test animals. Two types of fibrin hydrogel materials were formed between the injured surface. Fibrin film 1 (FF1) differs from fibrin film 2 (FF2) in that FF2 was compressed to release some water. The results show that the wounds treated with either normal fibrin hydrogel material (FF1) or compressed fibrin hydrogel material (FF2) have no adhesions between the caecum surface and the parietal surface. The control group for Table 9 had no fibrin hydrogel material applied, resulting in level 3 (the most severe) adhesions between the caecum surface and the parietal surface.

Table 10 shows the anti-adhesion properties of hydrogel fibrin glue material. This hydrogel material polymerizes within the body of the animal upon application using a delivery device such as that shown in Figure 6. The fibrin hydrogel glue was applied directly to the wound on the caecum surface, as well as to the parietal surface. All animals that received this fibrin hydrogel glue treatment were free of adhesion. In a second trial, a pre-cast fibrin hydrogel material was positioned between injured surfaces. Using a pre-cast fibrin hydrogel material demonstrated significant anti-adhesion properties as well.

Table 9

| Animal | Product Applied | Result |
|---|---|---|
| Control Rat 1 | none | adhesion (level 3) |
| Control Rat 2 | none | adhesion (level 3) |
| Control Rat 3 | none | adhesion (level 3) |
| Control Rat 4 | none | adhesion (level 3) |
| Rat 1 | FF1 | no adhesion |
| Rat 2 | FF1 | no adhesion |
| Rat 3 | FF2 | no adhesion |
| Rat 4 | FF2 | no adhesion |

Table 10

| Group Type | Number of Individuals | Thrombin Concentration | Result |
|---|---|---|---|
| Control | 5 | - | adhesion (level 3) |
| Pre-cast Hydrogel Fibrin Film | 5 | 100 IU/ml | 20 % adhesion |
| Hydrogel Fibrin Glue | 5 | 100 IU/ml | 0 % adhesion |

**Claims**

1. A method for producing a fibrin hydrogel material, the method comprising the steps of:

   providing a fibrinogen reagent;

providing a phosphate buffer calcium chelating agent;

providing thrombin essentially free of calcium; and

mixing the fibrinogen, the phosphate buffer chelating agent, and thrombin to form the fibrin hydrogel material, wherein the fibrin hydrogel material comprises a fibrin material having a water content of at least 90% by weight of the material and whereby the material retains at least 80% of the water upon compression by a force of 156G.

2. The method of Claim 1 wherein the fibrinogen reagent comprises fibrinogen carried in a first diluent.

3. The method of Claim 2 wherein the first diluent is a phosphate buffered saline solution.

4. The method of Claim 3 wherein fibrinogen has a concentration from 1.5 mg/ml to 100 mg/ml.

5. The method of Claim 1 wherein the thrombin reagent comprises thrombin carried in a second diluent.

6. The method of Claim 5 wherein the second diluent is a phosphate buffered saline solution.

7. The method of Claim 1 wherein thrombin has a concentration from 1 IU/ml to 1000 IU/ml.

8. The method of Claim 1 wherein the fibrin hydrogel material can be fabricated into articles selected from the group consisting of films, tubes, and pellets.

9. The method of Claim 8 wherein the fibrin hydrogel material can be fabricated into articles using techniques selected from the group of extrusion, molding, and thermal forming.

10. The method of Claim 8 wherein the fibrin hydrogel material can be sterilized at a temperature below 0°C by gamma radiation, stored at a temperature below 0°C, and used upon demand.

11. The method of Claim 10 wherein the sterilization by gamma radiation is below -25°C, at a dosage of at least 25 kGy.

12. An ex-vivo method for delivering fibrin hydrogel to a surface comprising the steps of:

providing a liquid solution of fibrinogen;

providing a liquid solution of thrombin essentially free of calcium;

providing a liquid solution of a phosphate buffer calcium chelating agent;

providing a spray unit in fluid communication with the fibrinogen solution, the thrombin solution, and the phosphate buffer chelating agent, the spray unit being capable of atomizing the fibrinogen solution, the thrombin solution, and the phosphate buffer chelating agent into an aerosol with at least one energy source of a liquid energy, a mechanical energy, a vibration energy, and an electric energy;

spraying the fibrinogen solution onto the surface with the spray unit;

spraying the phosphate buffer chelating agent onto the surface with the spray unit;

spraying the thrombin solution onto the surface with the spray unit; and,

mixing the fibrinogen solution, the phosphate buffer chelating agent and the thrombin solution,

wherein the fibrin hydrogel comprises a fibrin material having a water content of at least 90% by weight of the material and whereby the material retains at least 80% of the water upon compression by a force of 156G.

13. The ex-vivo method of Claim 12 wherein the phosphate buffer chelating agent is PBS.

14. The ex-vivo method of Claim 12 wherein the step of spraying the fibrinogen solution onto the surface occurs simultaneously with the step of spraying the thrombin solution onto the surface.

15. The ex-vivo method of Claim 12 wherein the step of mixing the fibrinogen solution and the thrombin solution defines a substantially homogeneous mixture prior to making contact with the surface.

16. The ex-vivo method of Claim 12 wherein the step of spraying the fibrinogen solution onto the surface and the step of spraying the thrombin solution onto the surface occurs sequentially.

**Patentansprüche**

1. Verfahren zur Herstellung eines Fibrinhydrogelmaterials, wobei das Verfahren die Schritte umfaßt:

    das Bereitstellen eines Fibrinogenreagens;
    das Bereitstellen eines Phosphatpuffer-Calciumchelatisierungsmittels;
    das Bereitstellen von Thrombin, das im wesentlichen frei von Calcium ist; und
    das Mischen des Fibrinogens, des Phosphatpuffer-Chelatisierungsmittels und von Thrombin unter Bildung des Fibrinhydrogelmaterials, wobei das Fibrinhydrogelmaterial ein Fibrinmaterial mit einem Wassergehalt von mindestens 90 Gew.-%, bezogen auf das Material, umfaßt, und wobei das Material mindestens 80% des Wassers nach Kompression mittels einer Kraft von 156 G zurückbehält.

2. Verfahren gemäß Anspruch 1, wobei das Fibrinogenreagens Fibrinogen, getragen in einem ersten Verdünnungsmittel, umfaßt.

3. Verfahren gemäß Anspruch 2, wobei das erste Verdünnungsmittel eine Phosphat-gepufferte Salzlösung ist.

4. Verfahren gemäß Anspruch 3, wobei das Fibrinogen eine Konzentration von 1,5 mg/ml bis 100 mg/ml aufweist.

5. Verfahren gemäß Anspruch 1, wobei das Thrombinreagens Thrombin, getragen in einem zweiten Verdünnungsmittel, umfaßt.

6. Verfahren gemäß Anspruch 5, wobei das zweite Verdünnungsmittel eine Phosphat-gepufferte Salzlösung ist.

7. Verfahren gemäß Anspruch 1, wobei das Thrombin eine Konzentration von 1 IU/ml bis 1000 IU/ml ausweist.

8. Verfahren gemäß Anspruch 1, wobei das Fibrinhydrogelmaterial in Gegenstände, ausgewählt aus der Gruppe, bestehend aus Folien, Schläuche und Pellets, verarbeitet werden kann.

9. Verfahren gemäß Anspruch 8, wobei das Fibrinhydrogelmaterial unter Verwendung von Techniken, ausgewählt aus der Gruppe von Extrusion, Formen und Thermoformen, in Gegenstände verarbeitet werden kann.

10. Verfahren gemäß Anspruch 8, wobei das Fibrinhydrogelmaterial bei einer Temperatur von unterhalb 0°C mittels Gammastrahlung sterilisiert, bei einer Temperatur von unterhalb 0°C gelagert und nach Bedarf verwendet werden kann.

11. Verfahren gemäß Anspruch 10, wobei die Sterilisation mittels Gammastrahlung unterhalb von -25°C, bei einer Dosis von mindestens 25 kGy, ist.

12. *Ex-vivo*-Verfahren zum Befördern von Fibrinhydrogel auf eine Oberfläche, umfassend die Schritte:

    das Bereitstellen einer flüssigen Lösung von Fibrinogen;
    das Bereitstellen einer flüssigen Lösung von Thrombin, im wesentlichen frei von Calcium;
    das Bereitstellen einer flüssigen Lösung eines Phosphatpuffer-Calciumchelatisienrungsmittels;
    das Bereitstellen einer Sprüheinheit in fluider Verbindung mit der Fibrinogenlösung, der Thrombinlösung und dem Phosphatpuffer-Chelatisierungsmittel, wobei die Sprüheinheit befähigt ist, die Fibrinogenlösung, die Thrombinlösung und das Phosphatpuffer-Chelatisierungsmittel in ein Aerosol mit mindestens einer Energiequelle von einer flüssigen Energie, einer mechanischen Energie, einer Vibrationsenergie und einer elektrischen Energie zu atomisieren;
    das Sprühen der Fibrinogenlösung auf die Oberfläche mit der Sprüheinheit;
    das Sprühen des Phosphatpuffer-Chelatisierungsmittels auf die Oberfläche mit der Sprüheinheit;
    das Sprühen der Thrombinlösung auf die Oberfläche mit der Sprüheinheit und
    das Mischen der Fibrinogenlösung, des Phosphatpuffer-Chelatisierungsmittels und der Thrombinlösung,

    wobei das Fibrinhydrogel ein Fibrinmaterial mit einem Wassergehalt von mindestens 90 Gew.-%, bezogen auf das Material, umfaßt, und wobei das Material mindestens 80% des Wassers nach Kompression durch eine Kraft von 156G zurückbehält.

**EP 1 387 703 B1**

**13.** *Ex-vivo*-Verfahren gemäß Anspruch 12, wobei das Phosphatpuffer-Chelatisierungsmittel PBS ist.

**14.** *Ex-vivo*-Verfahren gemäß Anspruch 12, wobei der Schritt des Sprühens der Fibrinogenlösung auf die Oberfläche gleichzeitig mit dem Schritt des Sprühens der Thrombinlösung auf die Oberfläche erfolgt.

**15.** *Ex-Vivo*-Verfahren gemäß Anspruch 12, wobei der Schritt des Mischens der Fibrinogenlösung und der Thrombinlösung ein im wesentlichen homogenes Gemisch vor Inkontaktreten mit der Oberfläche definiert.

**16.** *Ex-Vivo*-Verfahren gemäß Anspruch 12, wobei der Schritt des Sprühens der Fibrinogenlösung auf die Oberfläche und der Schritt des Sprühens der Thrombinlösung auf die Oberfläche nacheinander erfolgt.

## Revendications

**1.** Procédé de production d'une matière à base d'hydrogel de fibrine, le procédé comprenant les étapes consistant à :

fournir un réactif de fibrinogène ;
fournir un agent chélateur de calcium tamponné par les phosphates ;
fournir une thrombine essentiellement exempte de calcium ; et
mélanger le fibrinogène, l'agent chélateur tamponné par les phosphates, et la thrombine pour former la matière à base d'hydrogel de fibrine, dans lequel la matière à base d'hydrogel de fibrine comprend une matière à base de fibrine ayant une teneur en eau d'au moins 90 % en poids de la matière et grâce à quoi la matière conserve au moins 80 % de l'eau après compression par une force de 156 G.

**2.** Procédé selon la revendication 1, dans lequel le réactif de fibrinogène comprend le fibrinogène porté dans un premier diluant.

**3.** Procédé selon la revendication 2, dans lequel le premier diluant est une solution saline tamponnée par les phosphates.

**4.** Procédé selon la revendication 3, dans lequel le fibrinogène a une concentration de 1,5 mg/ml à 100 mg/ml.

**5.** Procédé selon la revendication 1, dans lequel le réactif de thrombine comprend la thrombine portée dans un deuxième diluant.

**6.** Procédé selon la revendication 5, dans lequel le deuxième diluant est une solution saline tamponnée par les phosphates.

**7.** Procédé selon la revendication 1, dans lequel la thrombine a une concentration de 1 UI/ml à 1 000 UI/ml.

**8.** Procédé selon la revendication 1, dans lequel la matière à base d'hydrogel de fibrine peut être façonnée en des articles choisis dans le groupe constitué par des films, des tubes et des granulés.

**9.** Procédé selon la revendication 8, dans lequel la matière à base d'hydrogel de fibrine peut être façonnée en des articles en utilisant des techniques choisies dans le groupe de l'extrusion, du moulage et du façonnage thermique.

**10.** Procédé selon la revendication 8, dans lequel la matière à base d'hydrogel de fibrine peut être stérilisée à une température inférieure à 0°C par rayonnement gamma, stockée à une température inférieure à 0°C, et utilisée à la demande.

**11.** Procédé selon la revendication 10, dans lequel la stérilisation par rayonnement gamma s'effectue sous -25°C, à un dosage d'au moins 25 kGy.

**12.** Procédé *ex vivo* pour administrer un hydrogel de fibrine sur une surface, lequel procédé comprenant les étapes consistant à :

fournir une solution liquide de fibrinogène ;
fournir une solution liquide de thrombine essentiellement exempte de calcium ;

**17**

fournir une solution liquide d'un agent chélateur de calcium tamponné par les phosphates ;

fournir un dispositif pulvérisateur en communication liquide avec la solution de fibrinogène, la solution de thrombine, et l'agent chélateur tamponné par les phosphates, le dispositif pulvérisateur étant capable de pulvériser la solution de fibrinogène, la solution de thrombine, et l'agent chélateur tamponné par les phosphates en un aérosol avec au moins une source énergétique d'une d'énergie liquide, d'une énergie mécanique, d'une énergie vibratoire, et d'une énergie électrique ;

pulvériser la solution de fibrinogène sur la surface avec le dispositif pulvérisateur ;

pulvériser l'agent chélateur tamponné par les phosphates sur la surface avec le dispositif pulvérisateur ;

pulvériser la solution de thrombine sur la surface avec le dispositif pulvérisateur ; et

mélanger la solution de fibrinogène, l'agent chélateur tamponné par les phosphates et la solution de thrombine,

dans lequel l'hydrogel de fibrine comprend une matière à base de fibrine ayant une teneur en eau d'au moins 90 % en poids de la matière et grâce à quoi la matière conserve au moins 80 % de l'eau après compression par une force de 156 G.

13. Procédé *ex vivo* selon la revendication 12, dans lequel l'agent chélateur tamponné par les phosphates est une solution PBS.

14. Procédé *ex vivo* selon la revendication 12, dans lequel l'étape consistant à pulvériser la solution de fibrinogène sur la surface se déroule simultanément à l'étape consistant à pulvériser la solution de thrombine sur la surface.

15. Procédé *ex vivo* selon la revendication 12, dans lequel l'étape consistant à mélanger la solution de fibrinogène et la solution de thrombine définit un mélange sensiblement homogène avant de venir en contact avec la surface.

16. Procédé *ex vivo* selon la revendication 12, dans lequel l'étape consistant à pulvériser la solution de fibrinogène sur la surface et l'étape consistant à pulvériser la solution de thrombine sur la surface se déroulent séquentiellement.

**Fig. 1**

**Fig. 2**

**Fig. 3**

**Fig. 4**

**Fig. 5**

Fig. 6

# Delivery device : pressurized can

**Fig. 7**

FIRST
CHAMBER 2

RUPTURABLE
MEMBRANE 3

CHARGE 1o

SECOND
CHAMBER 1

**Fig. 8**

EP 1 387 703 B1

Fig. 9

23

**Fig. 10**